# EUROPÄISCHE PATENTSCHRIFT

(11) **EP 3 174 992 B1**
(45) Veröffentlichungstag und Bekanntmachung des Hinweises auf die Patenterteilung: **14.04.2021**
(21) Anmeldenummer: 15747135.0
(22) Anmeldetag: 29.07.2015
(51) Int. Cl.: C12P 13/02, B01D 71/56, C08F 290/06

(54) **VERFAHREN ZUR ENZYMKATALYSIERTEN HERSTELLUNG VON PRÄPOLYMEREN FÜR DIE HERSTELLUNG VON KUNSTSTOFFEN**
METHOD FOR ENZYME-CATALYZED PRODUCTION OF PREPOLYMERS FOR THE MANUFACTURE OF PLASTICS
PROCÉDÉ DE PRODUIRE DES PRÉPOLYMÈRES CATALYSÉE PAR UNE ENZYME POUR LA PRODUCTION DE MATIÈRES PLASTIQUES

(30) Priorität: 31.07.2014 DE 102014215081
(43) Veröffentlichungstag der Anmeldung: 07.06.2017
(73) Patentinhaber: Karlsruher Institut für Technologie, 76344 Eggenstein-Leopoldshafen (DE); BioPro Baden-Württemberg GmbH, 70184 Stuttgart (DE)
(72) Erfinder: SYLDATK, Christoph, 76135 Karlsruhe (DE); KINDERVATER, Ralf, 75931 Gechingen (DE)
(74) Vertreter: Gille Hrabal Partnerschaftsgesellschaft mbB Patentanwälte
(86) Internationale Anmeldenummer: PCT/EP2015/067358
(87) Internationale Veröffentlichungsnummer: WO 2016/016293

(56) Entgegenhaltungen:
- EP-A1- 2 738 198
- WO-A1-95/07996
- WO-A1-2006/058697
- WO-A1-2006/106138
- WO-A1-2006/122922
- WO-A2-2009/092793
- YOUNG T-H ET AL: "Morphology of crystalline Nylon-610 membranes prepared by the immersion-precipitation process: competition between crystallization and liquid-liquid phase separation", POLYMER, ELSEVIER SCIENCE PUBLISHERS B.V, GB, Bd. 40, Nr. 18, 1. August 1999 (1999-08-01), Seiten 5011-5021, XP004164968, ISSN: 0032-3861, DOI: 10.1016/S0032-3861(98)00738-1
- SHIH C-H ET AL: "Morphology of membranes formed by the isothermal precipitation of polyamide solutions from water/formic acid systems", JOURNAL OF APPLIED POLYMER SCIENCE 20050505 JOHN WILEY AND SONS INC. US, Bd. 96, Nr. 3, 5. Mai 2005 (2005-05-05), Seiten 944-960, XP002746284, DOI: 10.1002/APP.21545

## Beschreibung

### Einleitung:

Die vorliegende Erfindung betrifft ein Verfahren zur enzymkatalysierten Herstellung von Präpolymeren für die Herstellung von Kunststoffen, basierend auf einer enzymkatalysierten Polymerisation von Monomer- oder Oligomerverbindungen. Insbesondere betrifft die Erfindung entsprechende Verfahren zur enzymkatalysierten Herstellung von Präpolymeren mit polyamidischer Bindungsstruktur für die Herstellung Polyamid-basierter Kunststoffe.

### Hintergrund:

Der derzeitige industrielle Haupterzeugungsweg für Kunststoffe und Kunststoffartikel basiert nahezu ausschließlich auf herkömmlichen petrochemischen Industrieverfahren, worin unter Einsatz fossiler Rohstoffe in großen Verbundanlagen riesige Mengen chemischer Vorprodukte erzeugt werden, die dann in Monomere, Rohpolymere, Feinpolymere und die entsprechenden Vorstufen der Kunststoffverarbeitung wie Granulate, Folien und Halbzeuge verarbeitet werden, um dann formgebend in der Kunststoffindustrie zu Endprodukten oder Bauteilen geformt zu werden.

Insbesondere vor dem Hintergrund der zunehmenden Ressourcenverknappung und den damit einhergehenden wachsenden Herausforderungen auch im Bereich der industriellen Fertigung von Konsumgütern, insbesondere in der Kunststoff-Industrie, dem Umwelt- und Klimaschutz verstärkt Rechnung zu tragen, wächst das Interesse und Bedürfnis, neue Fertigungswege mit verbesserter Nachhaltigkeit zu entwickeln und zu etablieren. Dabei ist es gerade unter dem Aspekt der Ressourcenknappheit von erheblichem Interesse, alternative Verfahren zu entwickeln, die es ermöglichen, die Verwendung fossiler Ressourcen zu reduzieren oder sogar ganz zu vermeiden. Diese Art einer neuen, sanften Chemie erfordert es, Vorstufen aus wässrigen Lösungen verwenden zu können, ohne hohe Anforderungen an die Reinheit der Zielsubstanzen stellen zu müssen.

Aus den vorgenannten Gründen wächst das Interesse und der Bedarf nach alternativen Verfahren, worin in diesen großchemischen Ansätzen der Einsatz petrochemischer Rohstoffe reduziert werden kann, bzw. worin petrochemische Rohstoffe durch nachhaltigere Rohstoffe ersetzt werden können, um bei zunehmender Rohstoffverknappung alternative Rohstoffquellen und energieeffizientere Herstellverfahren bereitstellen und damit langfristig die Sicherung der industriellen Erzeugungswege gewährleisten zu können.

Eine unter ökologischen Gesichtspunkten verbesserte Verfahrensführung sowie auch die Bereitstellung neuer Verfahren zur Herstellung nachhaltigerer Kunststoffe, sowie insbesondere auch die Herstellung biobasierter Kunststoffe auf Basis nachwachsender Rohstoffe, stellen dabei wichtige Aspekte dar.

### Stand der Technik:

Grundsätzlich sind Polymerisationsverfahren wie z.B. die Polykondensation zur Herstellung von Polymeren für die Kunststoffindustrie bekannt und in den üblichen in der Großindustrie angewendeten petrochemischen Verfahren der chemischen Polymerisation erfolgt die Verfahrensführung in organischen Lösungsmitteln oder in Salzschmelzen bzw. unter Verwendung aufwändiger wasserfreier Reaktorsysteme oder unter Anwendung azeotroper Destillation.

Nachteilig an diesen Verfahren ist einerseits die unter ökonomischen Gesichtspunkten aufwändige technische Verfahrensführung in komplexen Reaktorsystemen sowie andererseits die Notwendigkeit der hohen Reinheit der Vorstufen sowie der Abtrennung der organischen unpolaren Lösungsmittelsysteme und der damit verbundenen Notwendigkeit der Entsorgung bzw. des Recyclings. Die Erfinder der vorliegenden Erfindung haben nun ein neues Polymerisationsverfahren zur Herstellung von Präpolymeren, die für die Herstellung von Kunststoffen geeignet sind, gefunden, worin eine Polymerisation von geeigneten, in wässriger Lösung vorliegenden Monomeren und/oder Oligomeren mittels enzymkatalysierter Polymerisation zu den entsprechenden Präpolymeren erfolgt, die aus der wässrigen Reaktionslösung ausgefällt werden.

Dabei ist das erfindungsgemäße Verfahren insbesondere auch geeignet für die enzymkatalysierte Herstellung von Präpolymeren aus z.B. biotechnologisch hergestellten Monomeren oder Oligomeren, um daraus biobasierte Kunststoffe herzustellen, die über klassische petrochemische Syntheserouten reaktionstechnisch nur über viele Stufen und somit wirtschaftlich nicht sinnvoll herzustellen sind.

Grundsätzlich sind Verfahren zur Herstellung von Biokunststoffen auf Basis vollständig oder teilweise biobasierter Polymere, worin verstärkt die fossile Rohstoffbasis etablierter Standardmethoden durch eine erneuerbare Rohstoffbasis ersetzt wird, bereits bekannt. Beispiele hierfür sind z.B. biobasiertes Polyethylen (Bio-PE), Polypropylen (Bio-PP), Polyester und andere biobasierte Polymere. Darin werden insbesondere auch bereits biotechnologisch, beispielsweise fermentativ, erzeugte Polymere (Präpolymere), wie insbesondere fermentativ erzeugte Polyester, hergestellt und in der Herstellung von Biokunstoffen eingesetzt. Ein Beispiel für ein rekombinant hergestelltes Diaminopentan ist z.B. bekannt aus der WO 2009/092793, worin ein fermentativ hergestelltes Diaminopentan (DAP) aus einer DAP-haltigen Fermentationsbrühe isoliert wird, indem die Fermentationsbrühe alkalisiert, thermisch behandelt und anschließend das DAP mittels eines organischen Lösungsmittels extrahiert und schließlich aus der abgetrennten organischen Phase isoliert wird.

EP 2 738 198 A1 betrifft Verfahren zur enzymatischen Synthese von Polyamiden in wässrigen Mini-Emulsionen.

WO 2006/058697 A1 betrifft Verfahren zur Herstellung wässriger Polyamid-Dispersionen durch enzymkatalysierte Polykondensationsreaktion einer Diaminverbindung und einer Dicarbonsäureverbindung in wässrigem Medium.

WO 2006/122922 A1 betrifft Verfahren zur Herstellung wässriger Polymerdispersionen durch Umsetzung einer Diolverbindung und einer Dicarbonsäureverbindung in Anwesenheit eines Enzyms und eines Dispergiermittels zu einem Polyester.

WO 2006/106138 A1 betriff Verfahren zur Herstellung wässriger Polymerdispersionen durch Umsetzung einer Diaminverbindung und einer Dicarbonsäureverbindung in Anwesenheit eines Enzyms und eines Dispergiermittels zu einem Polyamid.

WO 95/07996 betrifft Verfahren zur Herstellung von Adipinsäure aus Kohlenstoffquellen auf Basis von Biomasse.

Young et al. "Morphology of crystalline Nylon-610 membranes prepared by the immersion-precipitation process: competition between crystallization and liquidliquid phase separation", Polymer Band 40, Nr. 18, Seiten 5011-5021, 1999 und Shih et al. "Morphology of membranes formed by the isothermal precipitation of polyamide solutions from water/formic acid systems", Journal of Applied Polymer Science Band 96, Nr. 3, Seiten 944-960, 2005 beschreiben Verfahren zur Herstellung von Nylon-Membranen durch isotherme Immersions-Präzipitation von Polyamidlösungen in Ameisensäure-Wasser Bädern.

Die WO 2013/044076 A1 beschreibt die fermentative Herstellung von Acrylsäure und anderen Carbonsäureverbindungen.

Der Einsatz biotechnologischer Verfahren zur Herstellung von Polymeren ist dabei insbesondere in Hinblick auf die Verfahrensökonomie und den Zugang zu bisher aus der Petrochemie nur schwer darstellbaren Kunststoffen mit neuen Produkteigenschaften von besonderem Interesse.

Grundsätzlich ist das Prinzip der enzymkatalysierten Polymerisiation bzw. der enzymatischen Synthese von Oligomeren bereits bekannt. So wird beispielsweise in der Dissertation von M. Andre ("Chemoenzymatische Herstellung von Peptiden und Acylpeptiden, spektralphotometrische, chromatografische und MALDI-ToF/MS Analysen der Produkte sowie Charakterisierung der tensidischen Eigenschaften"; 2012) die enzymatische Synthese von Di- und Oligopeptiden und die nachfolgende Synthese von acylierten Oligopeptiden zum Einsatz als Tenside beschrieben.

Außerdem wurden enzymkatalysierte Polymerisationsverfahren auch im Bereich der Herstellung von Oligomeren, die für die Herstellung von Biokunststoffen in Betracht kommen, bereits beschrieben. So erwähnt ein Übersichtsartikel von Gübitz und Paulo ("New substrates for reliable enzymes: enzymatic modification of polymer"; Current Opinion in Biotechnology, 2003, 14: 577-582) verschiedene Ansätze zur enzymkatalysierten Synthese von natürlichen und synthetischen Polymeren.

In der Dissertation von J. Duwensee ("Lipasen-katalysierte Polykondensation in wasserhaltigen Reaktionssystemen"; 2008) wird insbesondere eine Methode zur Herstellung von Polyestern zur Verwendung z.B. als Verpackungsmaterial oder in der Medizintechnik mittels Lipase-katalysierter Polymerisationsreaktion beschrieben.

Auch die Publikationen von Hilterhaus et al. ("Reactor Concept for Lipase-Catalyzed Solvent-Free Conversion of Highly Viscous Reactants Forming Two-Phase Systems"; Organic Process Research & Development, 2008, 12, 618-625) und Korupp et al. ("Scaleup of Lipase-Catalyzed Polyester Synthesis"; Organic Process Research & Development, 2010, 14, 1118-1124) beschreiben Verfahren zur Herstellung von Polyester mittels Lipase-katalysierter Polymerisationsreaktion.

Die DE 10 2005 026 135 A1 beschreibt ein Verfahren zur Herstellung einer wässrigen Polymerdispersion unter enzymkatalysierter Umsetzung einer Hydroxycarbonsäureverbindung zu einem Polyester in Anwesenheit eines Dispergiermittels aus der Gruppe der Emulgatoren und Schutzkolloide.

Allen diesen bekannten Verfahren ist jedoch gemeinsam, dass die Polymerisationsreaktion zur Herstellung der Oligomere und der daraus erhältlichen Kunststoffe bisher in wasserfreiem bzw. unpolarem Umfeld durchgeführt wird. Dies wird beispielsweise durch Verwendung eines wasserfreien Reaktorsystems, azeotrope Destillation des entstehenden Wassers aus dem Reaktionsmedium oder durch eine Verfahrensführung in unpolaren organischen Lösungsmitteln bzw. unter Einsatz unpolarer Lösungsmittelbestandteile und entsprechend einer Verfahrensführung in einem mindestens zweiphasigen (binären) Lösungsmittelsystem, umfassend eine polare wässrige Phase und eine unpolare organische Phase, erreicht.

So wird in den von Gübitz und Paulo zusammengestellten Verfahren z.B. für die Synthese von phenolischen Polymeren und Acryl-Polymeren mit Oxidoreduktasen eine Mizellen-Lösung verwendet. In der Laccase-katalysierten Synthese von Polyacrylamid und Polynatriumacrylat wird der Zusatz oberflächenaktiver Mittel zur Bildung einer Emulsion erwähnt. Sowohl bei der Verwendung einer Mizellen-Lösung als auch im Fall der Herstellung einer Emulsion liegt jedoch im Reaktionsmedium ein zweiphasiges System aus polarer wässriger Phase und unpolarer Phase vor. Weitere darin erwähnte Verfahren zu weiteren synthetischen Polymeren betreffen lediglich deren Oberflächenmodifikationen (z.B. von Polyester, Polyamid oder Polyacrylonitril).

Die in der Dissertation von Duwensee (2008) beschriebenen Verfahren verwenden ausschließlich binäre Lösungsmittelsysteme aus einer organischen unpolaren Extraktionsphase und einer wässrigen (polaren) Reaktionsphase.

In den von Hilterhaus et al. und Korupp et al. beschriebenen Verfahren erfolgt die Synthese in einem wasserfreien Reaktorsystem.

Wie bereits vorstehend erwähnt, ist dies einerseits unter dem Aspekt einer ökonomischen sowie andererseits in Hinblick auf eine ökologische Verfahrensführung nachteilig.

Insbesondere wenn die bekannten und vorstehend beschriebenen Polymerisationsverfahren unter Einsatz biotechnologisch hergestellter Monomere oder Oligomere als Ausgangsstoffe durchgeführt werden sollen, ergibt sich das Problem, dass diese biotechnologisch hergestellten Ausgangsstoffe üblicherweise in wässrigen Reaktionsmedien anfallen und dann für den Einsatz in den bekannten Polymerisationsverfahren erst aufwändig in hoher Reinheit in ein wasserfreies Medium überführt werden müssen. Im Gegensatz dazu ist aufgrund der hohen Selektivität des Reaktionssystems im Verfahren der vorliegenden Erfindung eine hohe Reinheit der anfallenden Ausgangsstoffe nicht unbedingt notwendig.

Außerdem ist eine genaue Einstellung des Mischungsverhältnisses der Monomere, die bei der chemischen Reaktionsführung von entscheidender Bedeutung ist, bei der enzymatischen Polymerisationsreaktion gemäß der vorliegenden Erfindung nicht mehr notwendig.

Das neue enzymkatalysierte Polymerisationsverfahren, welches erfindungsgemäß in einer einphasigen (polaren) wässrigen Lösung durchgeführt wird, ist somit besonders vorteilhaft gegenüber den bekannten Verfahren, da nun hiermit erstmals die Möglichkeit besteht, mit Reaktionsmedien zu arbeiten, die frei von unpolaren Lösungsmitteln sind. Darüber hinaus ist das neue Polymerisationsverfahren auch besonders geeignet, wenn biotechnologisch hergestellte Monomere oder Oligomere als Ausgangsstoffe eingesetzt werden sollen, da hiermit die Möglichkeit besteht, die wässrigen Monomer-/Oligomer-haltigen Fermentationsüberstände nach Zellabtrennung ohne Notwendigkeit einer weiteren vorherigen Aufreinigung direkt in der Polymerisationsreaktion zur Herstellung der Präpolymere einzusetzen. Dadurch kann eine deutliche Reduzierung der Prozessschritte und damit eine verbesserte Verfahrenseffizienz und -ökonomie erreicht werden.

Insbesondere wurden bisher keine Verfahren zur enzymkatalysierten Herstellung biobasierter Präpolymere mit polyamidischer Bindungsstruktur oder deren Verwendung zur Herstellung sogenannter Biokunststoffe auf Basis biobasierten Polyamids beschrieben.

### Aufgabenstellung:

Die Aufgabe der vorliegenden Erfindung bestand darin, ein neues Verfahren zur Herstellung von Präpolymeren für die Herstellung von Kunststoffen bereitzustellen, welches die Nachteile der aus dem Stand der Technik bekannten Verfahren vermeidet. Außerdem sollte sich das neue Verfahren durch eine verbesserte Verfahrensökonomie auszeichnen. In einem weiteren Aspekt der Erfindung sollte das neue Verfahren geeignet sein, eine Verfahrensführung mit hoher Nachhaltigkeit zu ermöglichen und es sollte für die Herstellung von Biokunststoffen aus vollständig und/oder teilweise biobasierten Mono- oder Oligomeren auf Basis nachwachsender Rohstoffe geeignet sein. Insbesondere sollte dabei das neue Verfahren die Herstellung von Präpolymeren mit polyamidischer Bindungsstruktur für die Herstellung neuer Kunststoffe auf Basis biobasierten Polyamids ermöglichen.

### Beschreibung der Erfindung:

Der Gegenstand der Erfindung wird ausschließlich durch den Wortlaut der Ansprüche festgelegt.

Gegenstand der vorliegenden Erfindung ist ein Verfahren zur Herstellung von Präpolymeren für die Herstellung von Kunststoffen, worin eine oder mehrere unterschiedliche Monomer- und/oder Oligomerverbindungen einer Polymerisationsreaktion unterworfen werden, welche dadurch gekennzeichnet ist, dass die Polymerisationsreaktion in einer einphasigen (polaren) wässrigen Lösung unter Zugabe eines oder mehrerer die Polymerisationsreaktion katalysierender Enzyme erfolgt, wobei die einphasige homogen mit Wasser mischbare wässrige Lösung frei von sonstigen unpolaren Lösungsmittelbestandteilen ist, die geeignet sind eine unpolare Phase in dem wässrigen Reaktionsmedium zu bilden.

Dabei werden die in dem wässrigen Reaktionsmedium gelöst vorliegenden Monomer- und/oder Oligomerverbindungen als Ausgangsstoffe durch enzymkatalysierte Reaktion zu längerkettigen Polymeren umgesetzt, bis eine Kettenlänge erreicht ist, ab der die gebildeten Polymere als sogenanntes Präpolymer aus der wässrigen (polaren) Reaktionslösung ausfallen.

Darin bezeichnet ein erfindungsgemäßes "Präpolymer" im Unterschied zu einem Oligomer, welches als möglicher Ausgangsstoff zur Herstellung der Präpolymere verwendet werden kann, das in der Polymerisationsreaktion aus den Monomeren/Oligomeren gebildete Molekül (Polymer) mit einer solchen Kettenlänge, ab der das gebildete Molekül (Polymer), im Gegensatz zum Oligomer, aus der wässrigen Reaktionslösung als sogenanntes Präpolymer ausfällt und hierüber aus dem wässrigen Reaktionsgemisch abgetrennt werden kann, um späterhin in anschließenden Reaktionsschritten zu längeren linearen oder verzweigten Homooder Copolymeren, Polymerblends (Kunststoffen) umgesetzt zu werden. Die spezifische Kettenlänge, ab der das gebildete Polymer als Präpolymer aus der wässrigen Monomer-/Oligomer-haltigen Reaktionslösung ausfällt hängt dabei einerseits von der Art der verwendeten Ausgangsstoffe und den daraus erhältlichen Präpolymeren sowie andererseits von den spezifischen Reaktionsbedingungen wie z.B. Temperatur, pH-Wert oder Zusammensetzung des Reaktionsmediums ab.

Die erfindungsgemäß hergestellten Präpolymere können grundsätzlich Homo- oder Copolymere sein. Dabei ist der Begriff Homo-/Copolymer dem Fachmann grundsätzlich bekannt.

Dabei sind erfindungsgemäß besonders bevorzugte Präpolymere solche, die eine polyamidische (polyamid-artige) Bindungsstruktur aufweisen. Darin bezeichnet eine polyamidische bzw. polyamid-artige Bindungsstruktur eine Bindungsstruktur über ein Strukturelement der allgemeinen Formel

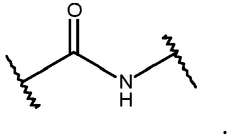

Auch bekannt, jedoch nicht zur Erfindung gehörig, sind Präpolymere, die eine polyester-artige Bindungsstruktur aufweisen. Darin bezeichnet eine polyester-artige Bindungsstruktur eine Bindungsstruktur über ein Strukturelement der allgemeinen Formel

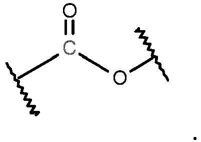

Der Begriff "Kunststoff" bezeichnet im üblichen Sinne einen polymeren Festkörper, der synthetisch oder halbsynthetisch aus den erfindungsgemäß gebildeten

Präpolymeren aufgebaut ist. Dabei können die erhältlichen Kunststoffe sowohl aus linearen als auch aus verzweigten und vernetzten Ketten bestehen.

Grundsätzlich wird bei Kunststoffen zwischen den drei großen Gruppen der Thermoplaste, der Duroplaste und der Elastomere unterschieden. Erfindungsgemäß bevorzugt sind thermoplastische und duroplastische Kunststoffe, wobei thermoplastische Kunststoffe besonders bevorzugt sind.

Das erfindungsgemäße Verfahren ist prinzipiell geeignet zur Herstellung von Präpolymeren, wie Polyestern (PES), umfassend z.B. Polybutylenterephtalat (PBT), Polyethylenterephthalat (PET), Polytrimethylenterephthalat (PTT), Polyethylennaphthalat (PEN), Polycarbonat (PC), und ungesättigtes Polyesterharz (UP) etc.; Polyamiden (PA), umfassend z.B. Polycaprolactam (Perlon; Polyamid 6)), Nylon (Polyamid 6.6; Polyhexamethylenadipinsäureamid), PA 69 (Hexamethylendiamin/Azelainsäure), PA 612 (Hexamethylendiamin/Dodecandisäure), PA 11 (11-Aminoundecansäure), PA 12 (Laurinlactam oder ω-Aminododecansäure), PA 46 (Tetramethylendiamin/Adipinsäure), PA 1212 (Dodecandiamin / Dodecandisäure), PA 6/12 (Caprolactam/Laurinlactam), PA 1010, etc.; Polyethylen (PE), umfassend Polyethylen hoher Dichte (PE-HD; HDPE), Polyethylen geringer Dichte (PE-LD; LDPE), lineares Polyethylen geringer Dichte (PE-LLD; LLDPE), hochmolekulares Polyethylen (PE-HMW); ultrahochmolekulares HDPE (PE-(UHMW), etc.; sowie Polypropylen (PP), Polystyrol (PS), Acrylnitril-Butadien-Styrol (ABS), Styrol-Acrylnitril (SAN), Polyoxymethylen (POM), Polymethacrylat (PMA), Polymethylmethacrylat (PMMA), Polyvinylchlorid (PVC), Polyphenylenether (PPE), Polyetheretherketon (PEEK), etc..

Erfindungsgemäß sind Präpolymere aus der Gruppe der Polyamide (PA) .

In dem erfindungsgemäßen Verfahren werden die erfindungsgemäßen Präpolymere durch katalytische Polymerisation aus geeigneten Monomer- und/oder Oligomerverbindungen gebildet. Darin können jeweils gleiche oder unterschiedliche Monomer- und/oder Oligomerverbindungen mit jeweils gleicher und/oder unterschiedlicher Kettenlänge miteinander umgesetzt werden. Das heißt, es kann beispielsweise eine Monomer- oder Oligomerverbindung mit einheitlicher Kettenlänge oder mit Bestandteilen unterschiedlicher Kettenlänge eingesetzt werden. Es ist auch möglich zwei oder mehrere unterschiedliche Monomer-und/oder Oligomerverbindungen miteinander umzusetzen, worin eine Monomer- bzw. Oligomerverbindung einheitliche Kettenlängen oder Bestandteile unterschiedlicher Kettenlänge aufweisen kann und worin die weitere(n) Monomer-und/oder Oligomerverbindung(en) ebenfalls einheitliche Kettenlängen oder Bestandteile unterschiedlicher Kettenlänge aufweisen können. Auch ist es möglich eine oder mehrere Monomerverbindungen, eine oder mehrere Oligomerverbindungen oder Monomer- mit Oligomerverbindungen miteinander umzusetzen.

Monomere oder Monomerverbindungen bezeichnen üblicherweise niedermolekulare reaktionsfähige Moleküle, die sich zu unverzweigten oder verzweigten Präpolymeren bzw. Polymeren zusammenschließen können. Monomere können Einzelsubstanzen, aber auch Gemische unterschiedlicher Verbindungen sein, wobei im ersten Fall Homopolymere und im zweiten Fall Copolymere gebildet werden. Oligomere oder Oligomerverbindungen bezeichnen üblicherweise Moleküle, die aus mehreren strukturell gleichen oder ähnlichen Einheiten (Monomeren) aufgebaut sind, aber gegenüber einem Präpolymer im Sinne der vorliegenden Erfindung noch in Wasser bzw. dem einphasigen wässrigen Reaktionsmedium löslich sind und somit als Reaktionspartner für eine enzymatische Reaktion in der wässrigen Reaktionslösung noch zur Verfügung stehen.

Im Rahmen der vorliegenden Erfindung sind bevorzugte Monomer- und Oligomerverbindungen ausgewählt aus der Gruppe umfassend Diamine, Carbonsäuren, wie insbesondere Hydroxycarbonsäuren, Di- und Tricarbonsäuren, Fettsäuren mit niederer, mittlerer und höherer Kettenlänge, Aminocarbonsäuren, Caprolactame, insbesondere Aminocaprolactame, Glucose, Lactone, Polyole, Diole, Glycole, Polyethylenglycole, Glycerin, (Di-, Tri-, Polyglycerin), Mono-, Di-, Tricarbonsäureester, etc., und jeweils Ester-Derivate davon, wie insbesondere Aminosäureester-Derivate, sowie Mischungen davon. Erfindungsgemäß besonders bevorzugte Monomer- und Oligomerverbindungen sind Diamine, Dicarbonsäuren, Aminocarbonsäuren, Caprolactam, insbesondere Aminocaprolactam sowie Carbonsäuren, insbesondere Citronensäure, Adipinsäure, Sebacinsäure und Bernsteinsäure.

Beispiele von Diaminverbindungen sind geradkettige oder verzweigte Diaminoalkane (H₂N-(C)ₙ-NH₂; mit n≥4), wie insbesondere C₄-C₂₈ Diaminoalkane, insbesondere C₄-C₂₀ Diaminoalkane, insbesondere C₄-C₁₂ Diaminoalkane, insbesondere C₄-C₁₀, Diaminoalkane, wie z.B. Diaminobutane, Diaminopentane, Diaminoheptane, Diaminoohexane, Diaminoheptane, Diaminoctane, Diaminononane, Diaminodecane, Diaminoundecane, Diaminododecane etc.; wie 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,11-Diaminoundecan, 1,12-Diaminododecan etc.. Umfasst sind ebenfalls die jeweiligen Konstitutionsisomere der genannten Diaminoalkane sowie solche, die gegebenenfalls mit weiteren Substituenten, wie z.B. Hydroxy, substituiert sein können. Besonders bevorzugt werden die Diaminoverbindungen ausgewählt aus der Gruppe, bestehend aus 1,4-Diaminobutan und 1,5-Diaminopentan.

Beispiele von Dicarbonsäuren sind C₂-C₂₈ Alkandisäuren, insbesondere C₂-C₁₆ Alkandisäuren und C₄-C₂₈-Alkandisäuren wie z.B. Oxalsäure (Ethandisäure), Malonsäure (Propandisäure), Bernsteinsäure (Butandisäure), Glutarsäure (Pentandisäure), Adipinsäure (Hexandisäure), Pimelinsäure (Heptandisäure), Korksäure, Suberinsäure (Octandisäure), Azelainsäure (Nonandisäure), Sebacinsäure (Decandisäure), Undecandisäure, Dodecandisäure (Decan-1,10-Dicarbonsäure), Brassylsäure (Tridecandisäure), Tetradecandisäure, Thapsiasäure (Hexadecandisäure) etc., sowie deren entsprechende Konstitutionsisomere; C₃-C₂₈ Alkendisäuren, insbesondere C₃-C₁₆ Alkendisäuren, sowie deren entsprechende Konstitutionsisomere sowie solche aus den vorgenannten Gruppen die mit einer oder mehreren, insbesondere einer oder zwei Hydroxy-, Keto- oder Amingruppen substituiert sein können wie z.B. Tartronsäure, Weinsäure, Äpfelsäure, α-Ketoglutarsäure, Oxalessigsäure, Phthalsäure, Isophthalsäure, Terephthalsäure, Glutaminsäure, Asparaginsäure, Maleinsäure, Fumarsäure, sowie Diphenylether-4,4-dicarbonsäure, Naphthalin-1,4-dicarbonsäure, Naphthalin-2,6-dicarbonsäure, und Hexahydroterephthalsäure. Umfasst sind ebenfalls die jeweiligen Konstitutionsisomere der genannten Carbonsäuren sowie solche, die gegebenenfalls mit weiteren Substituenten substituiert sein können. Besonders bevorzugt werden die Dicarbonsäuren ausgewählt aus 1,6-Hexandisäure und 1,10-Decandisäure.

Beispiele von Tricarbonsäuren sind z.B. Citronensäure, Isocitronensäure, Aconitsäure (1,2,3-Propentricarbonsäure), Carballylsäure (1,2,3-Propantricarbonsäure), Benzoltricarbonsäureen wie Trimesinsäure, Hemimellitsäure und Trimellitsäure. Beispiele von Hydroxycarbonsäuren umfassen Carbonsäuren, die mindestens eine Carboxygruppe als auch eine oder mehrere Hydroxygruppe(n) besitzen, wie z.B. α-, β- und γ-Hydroxycarbonsäuren. Beispiele von Hydroxycarbonsäuren sind, neben den vorstehend bereits genannten Hydroxydi- und -tricarbonsäuren, z.B. Glycolsäure, Mandelsäure, Milchsäure, Hydroxybuttersäure, Polyhydroxybuttersäure, Mevalonsäure, Gallussäure, 4-Hydroxybutansäure, 2-Hydroxybenzoesäure (Salicylsäure), 4-Hydroxybenzoesäure. Umfasst sind ebenfalls solche der vorgenannten Verbindungen, die gegebenenfalls mit weiteren Substituenten substituiert sein können.

Beispiele von Aminocarbonsäuren umfassen Carbonsäuren, die mindestens eine Carboxygruppe als auch eine oder mehrere Aminogruppe(n) besitzen. Beispiele sind C₁-C₂₀ Aminocarbonsäuren, insbesondere C₂-C₂₀ Aminocarbonsäuren, bevorzugt C₅-C₂₀-Aminocarbonsäuren wie z.B. α-, β- und γ-Aminosäuren wie z.B. die essentiellen Aminosäuren Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin; sowie Aminocarbonsäuren, die aus einer Amino-substituierten Mono-, Di- oder Tricarbonsäure, insbesondere wie vorstehend definiert, hervorgehen wie z.B. aus den vorgenannten Di- oder Tricarbonsäuren, die mit einer oder mehreren Amino-gruppen substituiert sind wie z.B. Aminoadipinsäure; sowie jeweils Aminosäureester-Derivate davon. Umfasst sind ebenfalls solche der vorgenannten Verbindungen, die gegebenenfalls mit weiteren Substituenten substituiert sein können.

Beispiele von Dicarbonsäureestern umfassen Ester der vorgenannten Dicarbonsäuren, die formal aus einer Dicarbonsäure, wie vorstehend definiert, und einem Alkohol oder Phenol zusammengesetzt sind. Umfasst sind außerdem die jeweiligen Konstitutionsisomere der genannten Dicarbonsäureester sowie solche, die gegebenenfalls mit weiteren Substituenten substituiert sein können.

Beispiele von Diolen umfassen C₂-C₂₈ Alkandiole, insbesondere C₂-C₁₆ Alkandiole wie z.B. 1,2-, 1,3-, 1,4-Alkandiole etc., beispielsweise die entsprechenden Ethan-, Propan-, Butandiole wie z.B. 1,2-Ethandiol (Ethylenglykol), 1,2- Propandiol (Propylenglykol), 1,3-Propandiol (1,3-Propylenglykol), 1,2-Butandiol, 1,3-Butandiol, 1,4-Butandiol, 2,3-Butandiol, Neopentylglykol, etc. sowie deren entsprechende Konstitutionsisomere, sowie α,ω-Diole die durch Kondensation von Ethylenglycol entstehen wie z.B. Diethylenglycol, Triethylenglycol, Polyethylenglycol etc., sowie z.B. Diethylenglykol, Cyclohexandimethanol, 2,2-bis(4-hydroxyphenyl)propan und 2,2-bis(4-hydroxyethoxyphenyl)propan. Umfasst sind ebenfalls solche der vorgenannten Verbindungen, die gegebenenfalls mit weiteren Substituenten substituiert sein können.

Dabei sind im Sinne der vorliegenden Erfindung von den vorstehenden Definitionen jeweils mögliche stereoisomere Konfigurationen (Enantiomere, Diastereomere sowie deren Racemate; α-, β-, γ, D-, L-Konfigurationen) umfasst. Ebenfalls sind erfindungsgemäß Ester-Derivate der vorstehenden Verbindungsgruppen umfasst, die aufgrund der jeweiligen spezifischen funktionellen Gruppen geeignet sind, in dem erfindungsgemäßen Verfahren entsprechend eingesetzt zu werden. Umfasst sind außerdem solche der vorgenannten Verbindungen, die gegebenenfalls mit weiteren Substituenten substituiert sein können, sofern dadurch die Funktionalität der reaktionsrelevanten Gruppen nicht beeinträchtigt ist.

Ganz besonders bevorzugt sind die Monomer- und Oligomerverbindungen ausgewählt aus der Gruppe der Diamine, der Dicarbonsäuren, der Aminocarbonsäuren sowie deren Ester-Derivate, der Hydroxycarbonsäuren, Caprolactame und/oder der Dicarbonsäureester.

In einer weiteren besonders bevorzugten Ausführungsform wird als Monomerverbindung eine oder mehrere Diaminverbindungen aus der Gruppe der Diaminoalkane, insbesondere C₄-C₁₀-Diaminoalkone, bevorzugter C₄-C₆-Diaminoalkane, eine oder mehrere Dicarbonsäuren, insbesondere C₆-C₂₈-Dicarbonsäuren, bevorzugter C₆-C₁₀-Dicarbonsäuren, eine oder mehrere Tricarbonsäuren, eine oder mehrere Aminocarbonsäuren, insbesondere C₂-C₂₀ Aminocarbonsäuren, bevorzugter C₅-C₂₀-Aminocarbonsäuren, eine oder mehrere Hydroxycarbonsäuren und/oder ein oder mehrere Caprolactame, insbesondere Aminocaprolactame, jeweils wie vorstehend definiert, ausgewählt.

In einer weiteren ganz besonders bevorzugten Ausführungsform wird als Monomerverbindung aus der Gruppe der Diaminoalkane 1,5-Diaminopentan, aus der Gruppe der Carbonsäuren Citronensäure, Adipinsäure, Sebacinsäure oder Bernsteinsäure, aus der Gruppe der Aminocarbonsäuren Aminoadipinsäure sowie Ester-Derivate davon, jeweils wie vorstehend definiert, und/oder Mischungen davon ausgewählt.

Erfindungsgemäß werden Monomer- und Oligomerverbindungen ausgewählt, die zur Herstellung von Präpolymeren mit polyamidischer Bindungsstruktur geeignet sind.

Darin ist es weiterhin bevorzugt, für die erfindungsgemäße Herstellung von Präpolymeren mit polyamidischer Bindungsstruktur die Monomer- und/oder Oligomerverbindung aus der Gruppe umfassend Diamine, Dicarbonsäuren, Aminocarbonsäuren und Ester-Derivate davon, Caprolactam sowie Aminocaprolactam auszuwählen. Dabei ist es einerseits bevorzugt, eine Mischung aus Dicarbonsäuren und Diaminen auszuwählen (die jeweils gleiche oder unterschiedliche Verbindungen mit jeweils gleicher oder unterschiedlicher Kettenlänge umfassen können), worin die beiden relevanten Gruppen der polyamidischen Bindungsstruktur, nämlich die Carbonyl- und die Amid-Gruppe, aus zwei unterschiedlichen Bausteinen, nämlich einerseits aus der Dicarbonsäure und andererseits aus der Diaminverbindung gebildet werden. Ebenfalls bevorzugt ist es, die Monomer- und/oder Oligomerverbindung aus der Gruppe der Aminocarbonsäuren, wie vorstehend definiert, zu wählen (worin eine oder Mischungen mehrerer Aminocarbonsäuren mit jeweils gleicher oder jeweils unterschiedlichen Kettenlängen enthalten sein können), worin quasi in einem einzigen Baustein beide relevanten Gruppen zur Bildung der polyamidischen Bindungsstruktur vorhanden sind.

Bezüglich der besonders bevorzugten Diamine, Carbonsäuren (insbesondere Dicarbonsäuren) und Aminocarbonsäuren kann auf die vorstehenden Definitionen verwiesen werden.

Zur Herstellung von nicht zur Erfindung gehörigen Präpolymeren mit polyester-artiger Bindungsstruktur werden die Monomer- und/oder Oligomerverbindungen bevorzugt ausgewählt aus den Gruppen der Di- und Tricarbonsäuren und Derivate davon, der Dialkohole (Diole) sowie Glycerin, alle jeweils wie vorstehend definiert. Bevorzugt ist eine Mischung umfassend eine oder mehrere Carbonsäureverbindungen, wie insbesondere Citronensäure, Adipinsäure, Sebacinsäure oder Bernsteinsäure und ein oder mehrere Diole, wie insbesondere 1,4-Butandiol und/oder Glycerin. Bezüglich der besonders bevorzugten Di- undTricarbonsäuren und Dialkohole (Diole) kann auf die vorstehenden Definitionen verwiesen werden.

Das erfindungsgemäße Verfahren ist durch eine enzymkatalysierte Polymerisation gekennzeichnet. Dabei können im Prinzip alle Enzyme eingesetzt werden, die geeignet sind die Umsetzung der ausgewählten Monomer- und/oder Oligomerverbindungen zu den gewünschten Präpolymeren zu katalysieren, insbesondere solche aus der Gruppe der Hydrolasen (Enzymklasse EC3), der Oxidoreduktasen (Enzymklasse EC1) und der Lyasen (Enzymklasse EC4). Besonders bevorzugt sind hier Enzyme aus der Gruppe der Hydrolasen (Enzymklasse EC3).

Beispiele geeigneter Enzyme aus der Gruppe der Hydrolasen umfassen z.B. Peptidasen (auch Proteasen, Proteinasen), Nukleasen, Phosphatasen, Glycosidasen, Esterasen, Lipasen, Lactamasen, Amidasen, (Amino)caprolactamasen, Polyamidasen, Carboxylesterase, Carboxypeptidasen, Amylasen etc. und Mischungen davon.

Beispiele geeigneter Enzyme aus der Gruppe der Oxidoreductasen umfassen z.B. Oxidasen, Dehydrogenasen und Reduktasen, wie Alkohol-Dehydrogenase, GlucoseOxidase, Aldehyd-Dehydrogenase, Dihydrofulat-Reduktase, Nitritreduktase, Ferredoxin-Nitritreduktase, Sulfit-Oxidase, Polyphenol-Oxidase, Katalase, Xanthin-Oxidase etc. und Mischungen davon.

Aus der Gruppe der Lyasen werden bevorzugt Decarboxylasen, ganz besonders bevorzugt L- bzw. D-Aminoadipinsäure-Decarboxylasen ausgewählt.

Erfindungsgemäß bevorzugte Enzyme aus der Gruppe der Hydrolasen sind z.B. Peptidasen Phosphatasen, Glycosidasen, Esterasen, Lipasen, Lactamasen, Amidasen, (Amino)caprolactamasen, Polyamidasen, Carboxylesterase, und Mischungen davon.

Erfindungsgemäß werden Enzyme ausgewählt, die zur Herstellung von Präpolymeren mit polyamidischer Bindungsstruktur geeignet sind.

Erfindungsgemäß werden zur Herstellung von Präpolymeren mit Polyamid-artiger Bindungsstruktur die Enzyme bevorzugt ausgewählt aus der Gruppe der Amidasen, Polyamidasen, Lactamasen und (Amino)caprolactamasen. Ganz besonders bevorzugt ist das Enzym die Protease "Subtilisin A" aus *Bacillus licheniformis.*

Zur Herstellung von nicht zur Erfindung gehörigen Präpolymeren mit Polyester-artiger Bindungsstruktur werden die Enzyme bevorzugt ausgewählt aus der Gruppe der Peptidasen und Proteasen.

Das erfindungsgemäße Verfahren ist speziell dadurch gekennzeichnet, dass die Polymerisationsreaktion in einer einphasigen (polaren) wässrigen Lösung durchgeführt wird. Dabei bezeichnet im Sinne der vorliegenden Erfindung eine wässrige Lösung eine hydrophile Reaktionslösung auf Wasserbasis, die im Wesentlichen frei von unpolaren organischen Lösungsmitteln oder Extraktionsmitteln ist. Darin ist die erfindungsgemäße wässrige Reaktionslösung insbesondere auch frei von sonstigen unpolaren (lipophilen) Lösungsmittelbestandteilen. Darin bezeichnet der Begriff "unpolare organische Lösungsmittel oder Extraktionsmittel" solche Lösungsmittel, die bekanntermaßen nicht mit Wasser mischbar sind. Der Begriff "sonstige unpolare Lösungsmittelbestandteile" bezeichnet solche Lösungsmittelzusätze, die geeignet sind eine unpolare Phase in dem wässrigen (polaren) Reaktionsmedium zu bilden, wie insbesondere Mizellen-bildende oder Vesikel- bzw. Liposom-, sowie Emulsion-bildende Substanzen. Somit kann die wässrige Lösung beispielsweise auch frei von Mizellen-, Vesikel- oder Liposomen-bildenden Substanzen und/oder frei von derartigen Mizellen, Vesikeln und Liposomen sowie frei von Emulsionen sein.

Eine Verfahrensführung in einphasigen wässrigen Reaktionssystemen kann in den bisher bekannten Verfahren nicht durchgeführt werden, da die Umsetzung der Monomere miteinander in der Regel unter Abspaltung von Wasser erfolgt und entsprechend eine geeignete Verschiebung des Reaktionsgleichgewichts auf die Seite der Polymere unter Wasserentzug durchgeführt wird.

In Verfahren der enzymkatalysierten Polymerisation, die aufgrund der Löslichkeit und Aktivität der Enzyme in Wasser zwingend in einem wässrigen Medium erfolgen muss, kann die Polymerisation unter Wasserabspaltung dennoch erreicht werden, da darin die gelösten monomeren oder oligomeren Ausgangsstoffe sozusagen in das Proteinmolekül (Enzym) hineindiffundieren und an dessen aktiven Zentrum eine derartige Ladungsverschiebung erfahren, dass die für die Polymerisationsreaktion notwendige und gewünschte Polymerisierung unter Wasserabspaltung erfolgen kann, quasi in dem von dem wässrigen Reaktionsmedium abgeschirmten Raum oder im Schutz des Proteinmoleküls.

Beim Arbeiten in rein wässrigen Systemen tritt allerdings der Effekt auf, daß die in der Polymerisationsreaktion gebildeten Polymere bereits ab einer vergleichsweise geringen Kettenlänge in Wasser unlöslich sind und in diesem rein wässrigen Reaktionsmedium ausfallen, womit sie einer weiteren Kettenverlängerung durch Polymerisation im Reaktionsansatz nicht mehr zur Verfügung stehen. In den bisher bekannten Verfahren ist ein derartiges Ausfallen der Präpolymere aus den genannten Gründen gerade unerwünscht und wird vermieden, indem ein zumindest zweiphasiges Lösungsmittelsystem verwendet wird, worin die in der polaren wässrigen Phase unlöslichen Präpolymere in der unpolaren Phase gelöst gehalten bleiben. Im Gegensatz dazu ist es im vorliegenden Verfahren jedoch explizit das Ziel, die gebildeten Polymere als sogenannte Präpolymere in dem wässrigen Reaktionsmedium auszufällen, um diese dann entweder daraus abzutrennen oder direkt in diesem Medium weiter zu verarbeiten, wie vorstehend und nachfolgend beschrieben.

Die wässrige Lösung, die der Polykondensationsreaktion unterworfen wird, besteht dabei erfindungsgemäß im Wesentlichen aus Wasser, wobei Anteile polarer Lösungsmittel zugesetzt werden können. Dabei wird der Zusatz an polaren Lösungsmitteln so gewählt, dass die Monomere und / oder die Oligomere im aktiven Zentrum des Enzyms bezogen auf ihre Ladung in einem für die Polymerisationsreaktion aktivierten Zustands (im Sinne einer geeigneten Ladungsverschiebung) vorliegen ohne dass dabei die Enzymaktivität eingeschränkt wird.

Erfindungsgemäß zugesetzte polare Lösungsmittel umfassen z.B. Methanol und Ethanol. Bevorzugt wird Ethanol zugesetzt.

Auch der Zusatz von pH-Wert regulierenden Stoffen, Puffersubstanzen oder die Variation der Salz- bzw. lonenkonzentration ist möglich. Über den geeigneten Zusatz derartiger Stoffe ist es möglich, das thermodynamische Gleichgewicht der enzymatisch katalysierten Polymerisationsreaktion von der Hydrolyse hin zur Synthese der Präpolymere günstig zu beeinflussen.

Ein einphasiges wässriges Lösungsmittelsystem im Sinne der vorliegenden Erfindung bezeichnet ein ausschließlich polares Lösungsmittelsystem, welches vollständig und homogen mit Wasser mischbar ist. Bevorzugt weist ein solches einphasiges wässriges Lösungsmittelsystem auch keinerlei unpolare Phasen oder Bereiche auf. Außerdem ist es möglich, über die Auswahl der technischen Verfahrensparameter, wie z.B. geeignete Temperatur- und Druckeinstellungen, die Auswahl geeigneter Reaktionszeiten sowie über die eingesetzten Enzymmengen und/oder die eingesetzten Monomer-/Oligomermengen (Überschuss / Unterschuss) einen Einfluss auf das thermodynamische Reaktionsgleichgewicht auszuüben und darüber das Verfahren günstig in die gewünschte Richtung der Synthese der Präpolymere zu beeinflussen. Dabei können entweder nur einzelne der vorgenannten Parameter oder mehrere der Parameter in beliebiger Kombination miteinander variiert und in geeigneter Weise eingestellt werden.

Die Polymerisationsreaktion ist ein im Prinzip bekanntes Reaktionsverfahren, welches nachfolgend beispielhaft in einem allgemeinen Reaktionsschema für das erfindungsgemäße Verfahren zur Herstellung von Präpolymeren mit polyamid-artiger sowie für Präpolymere mit polyester-artiger Bindungsstruktur skizziert wird. Darin bezeichnet jeweils
- R: Wasserstoff und/oder einen geeigneten Substituenten, welche bei einem Wert n>1 an den verschiedenen Positionen gleich oder unterschiedlich sein können,
- n: einen ganzzahligen Wert ≥ 1,
- m: einen ganzzahligen Wert ≥ 1.

Enzymkatalysierte Synthese von erfindungsgemäßen Präpolymeren mit polyamid-artiger Bindungsstruktur aus einer Mischung einer Diamin- und einer Dicarbonsäureverbindung:

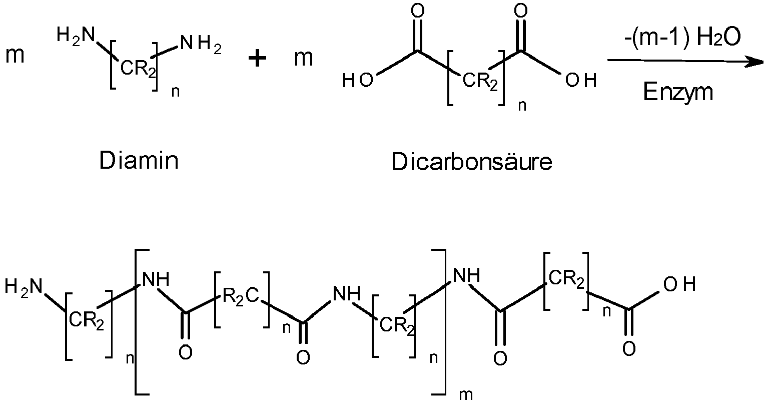

Enzymkatalysierte Synthese von erfindungsgemäßen Präpolymeren mit polyamid-artiger Bindungsstruktur aus einer Aminocarbonsäureverbindung:

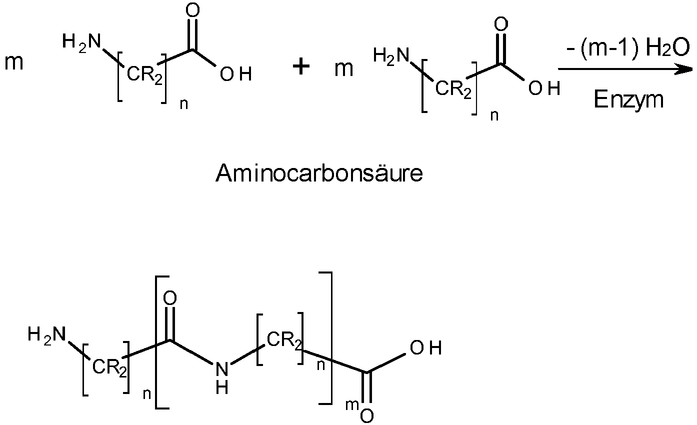

Enzymkatalysierte Synthese von nicht zur Erfindung gehörigen Präpolymeren mit polyester-artiger Bindungsstruktur aus einer Dicarbonsäure- und einer Diolverbindung:

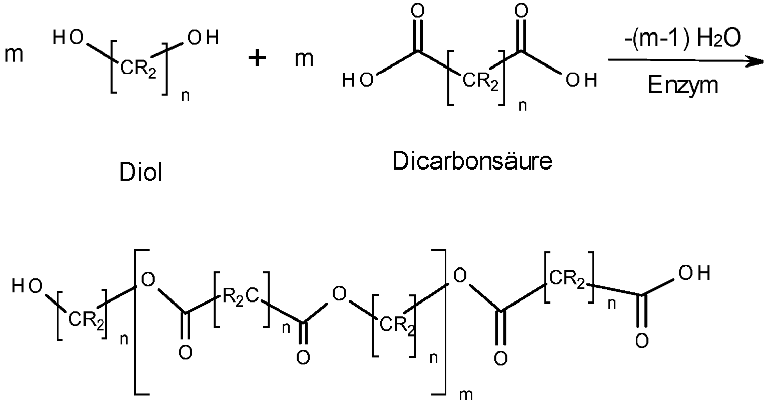

Enzymkatalysierte Synthese von nicht zur Erfindung gehörigen Präpolymeren mit polyester-artiger Bindungsstruktur aus einer Hydroxycarbonsäureverbindung:

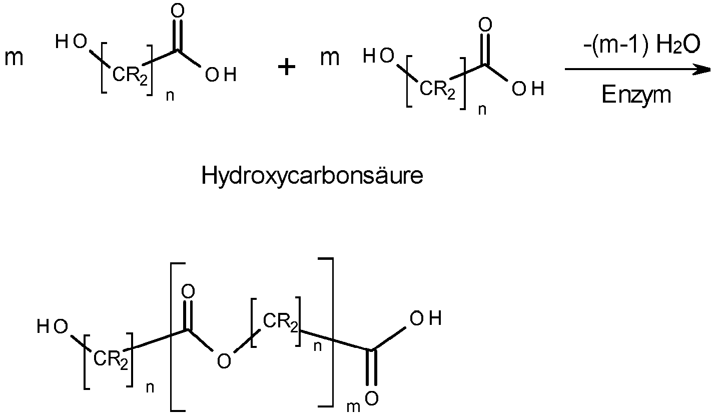

Die vorstehenden Darstellungen der Reaktionswege stellen dabei lediglich eine beispielhafte Skizzierung der grundsätzlichen Reaktionsprinzipien dar und wirken keineswegs einschränkend.

Die in dem erfindungsgemäßen Polymersationsverfahren gebildeten Präpolymere werden üblicherweise aus der wässrigen Reaktionslösung ausgefällt, mittels bekannter Verfahren wie beispielsweise Zentrifugation oder Filtration vom wässrigen

Überstand abgetrennt und gegebenenfalls in nachfolgenden Schritten zu thermoplastischen oder duroplastischen Kunststoffen weiterverarbeitet und gegebenenfalls mittels bekannten Verarbeitungsverfahren zu Kunststoffartikeln weiterverarbeitet, beispielsweise in Spinnverfahren oder thermoplastischen Formungsverfahren, insbesondere in Spritzgießverfahren, Guss- oder Extrusionsverfahren.

Somit umfasst das erfindungsgemäße Verfahren bevorzugt die Schritte:
a) Herstellung einer oder mehrerer Monomer- oder Oligomerverbindungen, beispielsweise durch Fermentation, enzymatische Umsetzung oder chemische Synthese, worin Fermentation und enzymatische Umsetzung bevorzugt sind,
b) Abtrennung derwässrigen Überstände mit den darin gelösten Monomer-oder Oligomerverbindungen,
c) Zugabe eines oder mehrerer die Polymerisationsreaktion der Monomer- oder Oligomerverbindungen katalysierender Enzyme zur wässrigen Lösung, die die Monomer- oder Oligomerverbindungen enthält,
d) Ausfällen der Präpolymere aus der wässrigen Reaktionslösung,
e) Abtrennen der ausgefällten Präpolymere, vorzugsweise durch Zentrifugation oder Filtration,
f) ggf. Weiterverarbeitung der Präpolymere zu Kunststoffen, und
g) ggf. Weiterverarbeitung der erhaltenen Kunststoffe zu Kunststoffartikeln, vorzugsweise in Spinnverfahren oder thermoplastischen Formungsverfahren, insbesondere in Spritzgießverfahren, Guss- oder Extrusionsverfahren.

Alternativ können auch Monomer- und/oder Oligomerverbindungen aus kommerziell erhältlichen Quellen, z.B. petrochemisch hergestellte Monomer-/Oligomerverbindungen, eingesetzt werden, wobei diese dann in Wasser gelöst werden und direkt dem Verfahrensschritt c) zugeführt werden.

In einem besonders bevorzugten Verfahren nach der vorliegenden Erfindung werden die eingesetzten Monomer- bzw. Oligomerverbindungen mittels biotechnologischer Verfahren, insbesondere mittels Fermentation oder enzymatischer Umsetzung erhalten. Dies ist besonders vorteilhaft unter dem Gesichtspunkt einer nachhaltigen Verfahrensführung, da dadurch die üblicherweise eingesetzten petrochemischen Rohstoffe durch nachhaltige biotechnologisch hergestellte Rohstoffe ersetzt werden können. Da mit dem erfindungsgemäßen Verfahren erstmals eine Verfahrensführung in wässrigem Reaktionsmedium möglich ist, ist der Einsatz fermentativ oder enzymatisch hergestellter Ausgangsverbindungen hier besonders geeignet. Üblicherweise fallen bei der fermentativen oder enzymatischen Herstellung der erfindungsgemäß eingesetzten Monomer- und Oligomerverbindungen dieselben im wässrigen Fermentationsüberstand bzw. wässrigen Reaktionsmedium gelöst an. Dies kann, ggf. nach Abtrennung der Zellen, mit den darin gelösten Monomer- bzw. Oligomerverbindungen in der Regel ohne Notwendigkeit einer weiteren Aufarbeitung unmittelbar weiterverarbeitet werden, indem direkt in diesem wässrigen Fermentationsüberstand oder Reaktionsmedium durch Zugabe der Enzyme die Polymerisationsreaktion initiiert wird. Ab einer bestimmten Kettenlänge fallen daraus die entsprechenden Präpolymere in der wässrigen Reaktionslösung aus, können wie vorstehend beschrieben abgetrennt und weiterverarbeitet werden. Besonders bevorzugt werden Monomer- bzw. Oligomerverbindungen verwendet, die mittels Fermentation erhalten werden und diese werden dann entsprechend bevorzugt direkt in dem wässrigen Fermentationsüberstand der Polykondensationsreaktion zugeführt.

Somit ist das erfindungsgemäße Verfahren besonders geeignet zur Herstellung von Präpolymeren aus biotechnologisch, insbesondere durch Fermentation, hergestellten Monomer- bzw. Oligomerverbindungen. In einer besonders bevorzugten Ausführungsform hiervon werden Präpolymere mit polyamid-artiger Bindungsstruktur hergestellt, worin als Monomerverbindung z.B. Diaminopentan als Diaminverbindung eingesetzt wird, welches durch Fermentation mittels eines rekombinanten Mikroorganismus erhältlich ist, wie z.B. mittels eines rekombinanten Bakteriums der Art *Corynebacterium glutamicum.*

Aufgrund des hierin möglichen Einsatzes biotechnologisch hergestellter Ausgangsstoffe mit hoher Nachhaltigkeit, als Ersatz zu fossilen Rohstoffen, werden im Sinne der vorliegenden Erfindung die daraus erhältlichen Präpolymere auch als biobasierte Präpolymere bezeichnet und die aus diesen biobasierten Präpolymeren hergestellten thermoplastischen und duroplastischen Kunststoffe werden als biobasierte Kunststoffe oder Biokunststoffe bezeichnet.

Die nach dem erfindungsgemäßen Verfahren erhältlichen Präpolymere können zur Herstellung von Kunststoffen, insbesondere thermoplastischen oder duroplastischen Kunststoffen, sowie daraus erhältlichen Kunststoffartikeln verwendet werden. Dabei können die nach dem erfindungsgemäßen Verfahren erhältlichen Präpolymere insbesondere zur Herstellung von Kunststoffen (Biokunststoffen) verwendet werden. Die nach dem erfindungsgemäßen Verfahren erhältlichen Präpolymere können zur Herstellung von Kunststoffen und Kunststoffartikeln verwendet werden, worin die Kunststoffartikel in Spinnverfahren, thermoplastischen oder duroplastischen Formungsverfahren, insbesondere in Spritzgieß-, Guss- oder Extrusionsverfahren, erhalten werden. Auch hier können die entsprechenden erfindungsgemäß herstellbaren biobasierten Präpolymere und Biokunststoffe verwendet werden.

Die nach dem erfindungsgemäßen Verfahren erhältlichen Präpolymere können zur Herstellung von Textilien, thermoplastischen Formteilen, Verpackungsmaterialien und Baustoffen, alle insbesondere auf Basis der erfindungsgemäß herstellbaren Biokunststoffe verwendet werden.

Durch die erfindungsgemäße Herstellung von Präpolymeren mit polyamidischer Bindungsstruktur sind entsprechend die daraus erhältlichen Kunststoffe (Biokunststoffe) und Kunststoffartikel zwangsläufig Polyamid-basierte Kunststoffe (Biokunststoffe) bzw. Kunststoffartikel.

### Beispiele

Im Folgenden wird die Erfindung an Hand eines Beispiels näher erläutert.

Zur Synthese von Polymerisationsprodukten auf Basis von Dicarbonsäuren und Diaminen wurden Hydrolasen aus der EC-Gruppe 3 verwendet. Es wurde die kommerziell erhältliche Protease "Subtilisin A" aus *Bacillus licheniformis* (Firma Megazyme; Bestellnummer: E-BSPRT) verwendet. Die Enzymstammlösung hatte 300 U / ml. Das pH-Optimum dieses Enzymes liegt bei pH 7 - 7.5, die pH-Stabilität liegt bei pH 5.5 - 10.0 und das Temperatur-Optimum bei 60°C.

Wenn nicht anders angegeben wurden alle verwendeten Lösungen in doppelt destilliertem Wasser (ddH₂O) angesetzt. Als Dicarbonsäuren wurden 1,6-Hexandisäure sowie 1,10-Decandisäure verwendet. Es wurde von 1,6-Hexandisäure eine 1 M Lösung bei 60°C hergestellt. 1,10-Decandisäure wurde entweder als 200 mM Lösung in 99,8 % Ethanol gelöst oder als 2,5 mM Lösung in Wasser angesetzt. Als Diamine dienten 1,4-Diaminobutan und 1,5-Diaminopentan. Davon wurde jeweils eine 400 mM Lösung in Wasser hergestellt.

Die enzymatische Synthese fand unter Schütteln in Reagenzgläsern mit Schraubverschluss bei 60° C und 1.500 rpm für 50-60 h in einem vortemperierten Thermoshaker ("Thermo Shaker Incubator" MS-100) statt. Dazu wurden 1000 µl der 1,4-Diaminobutan- und 62,2 µl 200 mM der Decandisäure (in Ethanol) mit 100 µl Enzymlösung versetzt. Um das Totalvolumen von 2,1 ml zu erreichen, wurde 969 µl ddH₂O zugegeben.

Alternativ wurde zu 1000 µl der 2,5 mM Decandisäurelösung 1000 µl 1,4-Diaminobutan oder 1,5-Diaminopentan und 100 µl Enzym zugegeben und mit ddH₂O aufgefüllt. Ebenso wurde zu 1000 µl 1,5-Diaminopentan oder 1,4-Diaminobutan 500 µl 1,6-Hexandisäurelösung und 100 µl Enzym zugegeben und mit ddH₂O aufgefüllt.

Der Anfangs-pH-Wert bei der Reaktion betrug pH 5.5 im Fall der Hexandisäure und pH 10.0 im Fall der Decandisäure.

Zur anschließenden Analyse wurden die Syntheseansätze am Rotationsverdampfer unter vermindertem Druck bei 50 mbar bei 60 Grad Celsius eingedampft. Von den eingedampften Syntheseansätzen wurden je 5-8 mg eingewogen und in ca. 1,5 ml HFIP-Lösung (99,9 % Hexafluorisopropanol, 0,1 wt % Kaliumtrifluoracetat) durch Rühren für mehrere Stunden wieder gelöst und anschließend filtriert (PTFE-Membran; 0,2 µm). Die Molekülmasse (Mn und MW) sowie die Dispersität der synthetisierten Produkte wurden mittels HFIP-Gel-Permeations-Chromatographie (HFIP-GPC) bestimmt. Je nach Syntheseansatz entstanden dabei sowohl Präpolymere mit kleineren Massen von ca. 700 - 1.300 Dalton sowie große Polymere mit Massen zwischen 100.000 und 300.000 Da; die Dispersität lag jeweils unter 1,25. Zur weiteren Analyse wurden die eingedampften Syntheseansätze in ca. 200 µl HFIP-Lösung wieder gelöst und in einem Überschuss kaltem Methanol gefällt. Gebildete Produkte fielen aus; der Überstand mit den Edukten wurde verworfen. Die Proben wurden getrocknet und nochmals mittels HFIP-GPC sowie mittels IR-Spektroskopie untersucht, wobei vor allem die kurzen Kettenlängen der Präpolymere von 700 - 1300 Da bestätigt wurden.

## Patentansprüche

1. Verfahren zur Herstellung von Präpolymeren für die Herstellung von Kunststoffen, worin eine oder mehrere Monomer- oder Oligomerverbindungen einer Polykondensationsreaktion unterworfen werden, welches **dadurch gekennzeichnet ist, dass** das Präpolymer eine polyamidische Bindungsstruktur aufweist und die Polykondensationsreaktion in einer einphasigen homogen mit Wasser mischbaren wässrigen Lösung unter Zugabe eines oder mehrerer die Polymerisationsreaktion katalysierender Enzyme erfolgt, wobei die einphasige homogen mit Wasser mischbare wässrige Lösung frei von sonstigen unpolaren Lösungsmittelbestandteilen ist, die geeignet sind eine unpolare Phase in dem wässrigen Reaktionsmedium zu bilden.

2. Verfahren nach Anspruch 1, worin die Präpolymere aus der einphasigen homogen mit Wasser mischbaren wässrigen Reaktionslösung ausgefällt werden und anschließend daraus abgetrennt und zu Kunststoffen weiter verarbeitet werden,

3. Verfahren nach einem der vorhergehenden Ansprüche, zur Herstellung von Präpolymeren für die Herstellung von Kunststoffen die aus der Gruppe der thermoplastischen und duroplastischen Kunststoffe ausgewählt sind,

4. Verfahren nach einem der vorhergehenden Ansprüche, worin das Präpolymer Polyamid (PA) ist,

5. Verfahren nach einem der vorhergehenden Ansprüche, worin die Monomer-und/oder Oligomerverbindungen ausgewählt sind aus der Gruppe umfassend Diamine, Carbonsäuren, wie insbesondere Hydroxycarbonsäuren, Di- und Tricarbonsäuren, Aminocarbonsäuren, Caprolactame, insbesondere Aminocaprolactame, und jeweils Ester-Derivate und Mischungen davon.

6. Verfahren nach Anspruch 5, worin die Diamine ausgewählt werden aus C₄-C₆-Diaminoalkanen, die Dicarbonsäuren ausgewählt werden aus C₆-C₁₀-Dicarbonsäuren, die Aminocarbonsäuren ausgewählt werden aus C₅-C₂₀-Aminocarbonsäuren und jeweils Ester-Derivaten davon, und worin Aminocaprolactam aus der Gruppe der Caprolactame ausgewählt wird.

7. Verfahren nach einem der vorhergehenden Ansprüche, worin die Enzyme ausgewählt sind aus der Gruppe der Hydrolasen, Oxidoreduktasen und Lyasen, bevorzugt aus der Gruppe der Hydrolasen.

8. Verfahren nach einem der vorhergehenden Ansprüche, worin als Monomerverbindung aus der Gruppe der Diaminoalkane 1,5-Diaminopentan, aus der Gruppe der Carbonsäuren Citronensäure, Adipinsäure, Sebacinsäure oder Bernsteinsäure, aus der Gruppe der Aminocarbonsäuren Aminoadipinsäure sowie jeweils Ester-Derivate davon, und/oder Mischungen davon ausgewählt werden und worin die Enzyme aus der Gruppe der Hydrolasen ausgewählt sind aus Amidasen, Polyamidasen, Lactamasen und (Amino)caprolactamasen.

9. Verfahren nach einem der vorhergehenden Ansprüche, worin
(i) das Präpolymer eine polyamidische Bindungsstruktur aufweist und
(ii) als Monomere oder Oligomere eine Mischung einer oder mehrerer Diaminverbindungen mit einer oder mehreren Dicarbonsäureverbindungen, eine oder mehrere Aminocarbonsäuren oder Ester davon oder Caprolactam, insbesondere Aminocaprolactam, und
(iii) als Enzym eine Hydrolase, vorzugsweise eine Polyamidase oder eine (Amino)caprolactamase eingesetzt werden.

10. Verfahren nach einem der vorhergehenden Ansprüche, worin die Monomer-oder Oligomerverbindungen durch Fermentation hergestellt werden, bevorzugt durch Fermentation mittels eines rekombinanten Mikroorganismus.

11. Verfahren nach einem der vorhergehenden Ansprüche, umfassend die Schritte
a) Herstellung von einer oder mehreren Monomer- oder Oligomerverbindungen, vorzugsweise durch Fermentation oder enzymatische Umsetzung,
b) Abtrennung der wässrigen Überstände mit den darin gelösten Monomer-oder Oligomerverbindungen,
c) Zugabe eines oder mehrerer die Polymerisationsreaktion der Monomer-oder Oligomerverbindungen katalysierender Enzyme zur wässrigen Lösung, die eine oder mehrere Monomer- oder Oligomerverbindungen enthält,
d) Ausfällen der Präpolymere aus der wässrigen Reaktionslösung,
e) Abtrennen der ausgefällten Präpolymere, vorzugsweise durch Zentrifugation oder Filtration,
f) ggf. Weiterverarbeitung der abgetrennten Präpolymere zu Kunststoffen, und
g) ggf. Weiterverarbeitung der erhaltenen Kunststoffe zu Kunststoffartikeln, vorzugsweise in Spinnverfahren oder thermoplastischen oder duroplastischen Formungsverfahren, insbesondere in Spritzgieß-, Guss- oder Extrusionsverfahren.

## Claims

1. A process for preparing prepolymers for the production of plastics, wherein one or more monomer or oligomer compounds are subjected to a polycondensation reaction, which is **characterized in that** the prepolymer has a polyamide-type bonding structure and the polycondensation reaction is carried out in a single phase aqueous solution, which is homogeneously miscible with water, with the addition of one or more enzymes catalyzing the polymerization reaction, said single phase, homogeneously with water miscible aqueous solution being free of other non-polar solvent components, which are suitable to form a non-polar phase in the aqueous reaction medium.

2. The process of claim 1, wherein the prepolymers are precipitated from the single-phase aqueous reaction solution and then separated therefrom and further processed into plastics.

3. The process according to one of the preceding claims for the preparation of prepolymers for the production of plastics which are selected from the group of thermoplastics and thermosetting plastics.

4. The process according to one of the preceding claims, wherein the prepolymer is polyamide (PA).

5. The process according to one of the preceding claims wherein the monomer and / or oligomer compounds are selected from the group comprising diamines, carboxylic acids, in particular hydroxycarboxylic acids, di- and tricarboxylic acids, amino carboxylic acids, caprolactams, particularly aminocaprolactams, and ester derivatives and mixtures thereof, respectively.

6. The process according to claim 5 wherein the diamines are selected from C₄-C₆- diaminoalkanes, the dicarboxylic acids are selected from C₆-C₁₀-dicarboxylic acids, the amino carboxylic acids are selected from C₅-C₂₀-amino carboxylic acids and ester derivatives thereof, respectively, and wherein the aminocaprolactam is selected from the group of caprolactams.

7. The process according to one of the preceding claims, wherein the enzymes are selected from the group of hydrolases, oxidoreductases and lyases, preferably from the group of hydrolases.

8. The process according to claim 5 wherein the monomer compound selected from the group of diaminoalkanes is 1,5-diaminopentane, from the group of carboxylic acids is citric acid, adipic acid, sebacic acid or succinic acid, from the group of amino carboxylic acids is aminoadipic acid and ester derivatives thereof, respectively and/or mixtures thereof, and wherein the enzyme from the group of hydrolases is selected from amidases, polyamidases, lactamases and (amino)caprolactamases.

9. The process according to one of the preceding claims, wherein
(i) the prepolymer has a polyamide-type bonding structure and
(ii) as monomers or oligomers a mixture of one or more diamine compounds with one or more dicarboxylic acid compounds, one or more amino carboxylic acids or esters thereof, or caprolactam, in particular aminocaprolactam, and
(iii) as enzyme a hydrolase, preferably a polyamidase or (amino)caprolactamase is used.

10. The process according to one of the preceding claims, wherein the monomer or oligomer compounds are prepared by fermentation, preferably by fermentation using a recombinant microorganism.

11. The process according to one of the preceding claims, comprising the steps
a) preparing one or more monomer or oligomer compounds, preferably by fermentation or enzymatic reaction,
b) separating the aqueous supernatants with the monomer or oligomer compounds dissolved therein,
c) adding one or more enzymes catalyzing the polymerization reaction of the monomer or oligomer compounds to the aqueous solution containing one or more monomer or oligomer compounds,
d) precipitating the prepolymers from the aqueous reaction solution,
e) separating the precipitated prepolymers, preferably by centrifugation or filtration,
f) optionally further processing of the separated prepolymers to plastics, and
g) optionally further processing of the resulting plastics into plastic articles, preferably in spinning processes or thermoplastic or thermosetting molding processes, in particular in injection molding, casting or extrusion processes.

## Revendications

1. Procédé de préparation de prépolymères en vue de la préparation de matières synthétiques, dans lequel un ou plusieurs composés monomères ou oligomères sont soumis à une réaction de polycondensation, lequel est **caractérisé en ce que** le prépolymère présente une structure de liaison polyamidique et la réaction de polycondensation a lieu dans une solution aqueuse homogène monophasée mélangeable à de l'eau avec ajout d'un ou de plusieurs enzymes catalysant la réaction de polymérisation, selon lequel la solution aqueuse homogène monophasée mélangeable à de l'eau est exempte d'autres constituants dissolvants non polaires, qui sont susceptibles de former une phase non polaire dans le milieu réactionnel aqueux.

2. Procédé selon la revendication 1, dans lequel les prépolymères sont précipités hors de la solution réactionnelle aqueuse homogène monophasée mélangeable à de l'eau et en sont ensuite séparés et ultérieurement transformés en matières synthétiques.

3. Procédé selon l'une quelconque des revendications précédentes, pour la préparation de prépolymères en vue de la préparation de matières synthétiques choisies dans le groupe des matières synthétiques thermoplastiques et duroplastiques.

4. Procédé selon l'une quelconque des revendications précédentes, dans lequel le prépolymère est un polyamide (PA).

5. Procédé selon l'une quelconque des revendications précédentes, dans lequel les composés monomères et/ou oligomères sont choisis dans le groupe comprenant de la diamine, des acides carboxyliques, comme en particulier des acides hydroxycarboxyliques, des acides di- et tricarboxyliques, des acides aminocarboxyliques, du caprolactame, en particulier de l'aminocaprolactame, et respectivement des dérivés d'esters et des mélanges de ceux-ci.

6. Procédé selon la revendication 5, dans lequel les diamines sont choisies parmi des diaminoalcanes C₄-C₆, les acides dicarboxyliques sont choisis parmi des acides dicarboxyliques C₆-C₁₀, les acides aminocarboxyliques sont choisis parmi des acides aminocarboxyliques C₅-C₂₀ et respectivement des dérivés d'esters de ceux-ci, et dans lequel l'aminocaprolactame est choisi dans le groupe des caproiactames.

7. Procédé selon l'une quelconque des revendications précédentes, dans lequel les enzymes sont choisis dans le groupe des hydrolases, des oxidoréductases et des lyases, de préférence dans le groupe des hydrolases.

8. Procédé selon l'une quelconque des revendications précédentes, dans lequel comme composé monomère sont choisis dans le groupe des diaminoalcanes, du 1,5-diaminopentane, dans le groupe des acides carboxyliques, de l'acide citrique, de l'acide adipique, de l'acide sébacique ou de l'acide succinique, dans le groupe des acides aminocarboxyliques, des acides aminoadipiques, ainsi que respectivement de dérivés d'esters de ceux-ci, et/ou de mélanges de ceux-ci, et dans lequel les enzymes du groupe des hydrolases sont choisis parmi des amidases, des polyamidases, des lactamases et des (amino)caprolactamases.

9. Procédé selon l'une quelconque des revendications précédentes, dans lequel
(i) le prépolymère présente une structure de liaison polyamidique et
(ii) sont utilisés comme monomères ou oligomères, un mélange d'un ou de plusieurs composés de diamine avec un ou plusieurs composés d'acide dicarboxylique, un ou de plusieurs acides aminocarboxyliques ou des esters de ceux-ci, ou du caprolactame, en particulier de l'aminocaprolactame, et
(iii) comme enzyme, une hydrolase, de préférence une polyamidase ou une (amino)caprolactamase.

10. Procédé selon l'une quelconque des revendications précédentes, dans lequel les composés monomères ou oligomères sont préparés par fermentation, de préférence par fermentation au moyen d'un microorganisme recombinant.

11. Procédé selon l'une des revendications précédentes, comprenant les étapes
a) de préparation d'un ou de plusieurs composés monomères ou oligomères, de préférence par fermentation ou réaction enzymatique,
b) de séparation des surnageants aqueux avec les composés monomères ou oligomères qui y sont dissous,
c) d'addition d'une ou de plusieurs enzymes catalysant la réaction de polymérisation des composés monomères ou polymères à la solution aqueuse, qui contient un ou plusieurs composés monomères ou polymères,
d) de précipitation des prépolymères hors de la solution réactionnelle aqueuse,
e) de séparation des prépolymères précipités, de préférence par centrifugation ou filtration,
f) le cas échéant, de traitement ultérieur des prépolymères séparés en matières synthétiques, et
g) le cas échéant, de traitement ultérieur des matières synthétiques obtenues en articles en matières synthétiques, de préférence dans un procédé de filage ou un procédé de formage thermoplastique ou duroplastique, en particulier dans un procédé de moulage par injection, de coulée ou d'extrusion.
